# EUROPEAN PATENT APPLICATION

(11) **EP 3 133 094 A1**
(43) Date of publication of application: **22.02.2017**
(21) Application number: 15780111.9
(22) Date of filing: 27.03.2015
(51) Int. Cl.: C08G 18/20, C08G 18/30, C09J 175/02

(54) **CURING AGENT FOR USE IN PRODUCTION OF POLYUREA RESIN, AND POLYUREA RESIN PRODUCTION METHOD USING SAME**

(30) Priority: 16.04.2014 JP 2014084842
(71) Applicant: Tosoh Corporation, Shunan-shi, Yamaguchi 746-8501 (JP)
(72) Inventor: TAKAHASHI Yoshihiro, Shunan-shi Yamaguchi 746-8501 (JP); KISO Hiroyuki, Shunan-shi Yamaguchi 746-8501 (JP)
(74) Representative: Vigand, Philippe
(86) International application number: PCT/JP2015/059746
(87) International publication number: WO 2015/159690

(57) **Abstract**

To provide a curing agent for producing a polyurea resin, which is capable of thermal compression molding, which will not cause odor and toxicity or environmental problems at the time of molding or of products, which has favorable adhesive strength, and which hardly causes pollution of other materials, and a method for producing a polyurea resin using it.

A curing agent for producing a polyurea resin, which comprises at least one diamine compound selected from the group consisting of compounds each represented by the following formula (1): 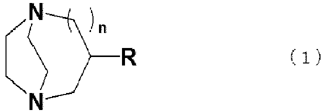 wherein n is 0 or 1, and R is a hydroxy group or hydroxyalkyl group having 0 to 2 carbon atoms, and at least one amine compound selected from the group consisting of N,N-dimethylaminoethanol, N,N-dimethylaminoisopropanol and N,N-dimethyl-N',N'-di(2-hydroxypropyl)propylenediamine.

## Description

### TECHNICAL FIELD

The present invention relates to a curing agent for producing a polyurea resin, and a method for producing a polyurea resin using it.

### BACKGROUND ART

A synthetic board has been used for e.g. a building material to be used for the deck, a terrace, a fence, a handrail, a column, a bench, etc. of a building, a core material of an inner lining of a door as a vehicle interior part, a core material of a panel on the floor of a luggage room, etc. Such a synthetic board is a synthetic material obtained by mixing small pieces of e.g. wood with an adhesive, followed by thermal compression molding, and for example, a wood synthetic board such as an oriented strand board (OSB), a particle board or a medium density fiberboard (MDF), a synthetic board formed of foam chips, a rubber ground product, paper, cloth or rice hull, a board formed of vegetable fibers, and a synthetic board obtained by forming an inorganic lightweight aggregate, have been known.

Further, a polyurethane foam sheet, a glass chopped strand mat, and a ceiling material for an automobile obtained by laminating e.g. a skin, followed by thermocompression bonding has been known.

Heretofore, as a general-use adhesive to be used for production of such a synthetic board or a ceiling material for an automobile, a urea resin, a urea melamine resin or a phenol resin has been mainly used, however, from a synthetic board or a ceiling material for an automobile using such a general-use adhesive, formaldehyde, which is one of substances causing sick house syndrome, is emitted.

Further, as an adhesive for such applications, use of a polyurea resin has been attempted.

A polyurea resin is a polymer compound having in its skeleton a urea linkage formed by an addition reaction of an amino group (NH₂) and an isocyanate group (NCO), and is free from emission of formaldehyde.

However, a tertiary amine compound used as a curing agent therefor is gradually emitted as a volatile amine from the polyurea resin and causes, for example, an odor by the volatile amine and discoloration or whitening of other materials (for example, the skin polyvinyl chloride or polycarbonate) e.g. for a ceiling material for an automobile. Further, the tertiary amine compound usually emits a strong odor and thereby remarkably deteriorates the working environment for production of a synthetic board or a ceiling material for an automobile, or causes an amine odor called "the smell of a new car".

To solve the problems of such a volatile tertiary amine compound, an amine compound having in its molecule a hydroxy group reactive with a polyisocyanate, a primary or secondary amino group, and the like in its molecule (generally called "a reactive amine catalyst") has been proposed (for example, Patent Documents 1 to 9).

According to the above Patent Documents, the above problems can be avoided since such an amine compound is fixed in the resin skeleton of a polyisocyanate compound as reacted with the polyisocyanate, however, such documents disclose use of the amine compounds as a polyurethane catalyst, and when the amine compound is used as a curing agent for producing a polyurea resin, reduction of the odor, and prevention of discoloration or whitening of other materials are not sufficiently achieved, and further improvement has been desired.

Further, a curing agent for producing a polyurea resin (for example, Patent Document 10) has been proposed, and improvement in reduction of the odor and prevention of discoloration or whitening of other materials has been brought about, however, further improvement has been required for commercial application.

### PRIOR ART DOCUMENTS

### PATENT DOCUMENTS

- Patent Document 1:: JP-A-46-4846
- Patent Document 2:: JP-B-61-31727
- Patent Document 3:: Japanese Patent No. 2971979
- Patent Document 4:: JP-A-63-265909
- Patent Document 5:: JP-A-2008-45113
- Patent Document 6:: U.S. Patent No. 4007140
- Patent Document 7:: JP-A-2003-260769
- Patent Document 8:: JP-A-2012-131839
- Patent Document 9:: JP-A-2012-149225
- Patent Document 10:: JP-A-2014-40587

### DISCLOSURE OF INVENTION

### TECHNICAL PROBLEM

Under these circumstances, the object of the present invention is to provide a curing agent for producing a polyurea resin, which is capable of thermal compression molding at the time of producing a synthetic board or at the time of producing a ceiling material for an automobile, which is free from the odor and toxicity or environmental problems at the time of the molding or of products, which has favorable adhesive strength, and which hardly causes pollution of other materials, and a method for producing a polyurea resin using it.

### SOLUTION TO PROBLEM

The present inventors have conducted extensive studies to achieve the above object and as a result, found the curing agent for producing a polyurea resin of the present invention and the method for producing a polyurea resin using it, and accomplished the present invention.

That is, the present invention provides the following curing agent for producing a polyurea resin and method for producing a polyurea resin.
[1] A curing agent for producing a polyurea resin, which comprises at least one diamine compound selected from the group consisting of compounds each represented by the following formula (1): 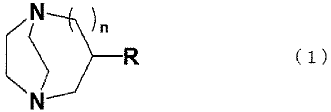 wherein n is 0 or 1, and R is a hydroxy group or hydroxyalkyl group having 0 to 2 carbon atoms, and at least one amine compound selected from the group consisting of N,N-dimethylaminoethanol, N,N-dimethylaminoisopropanol and N,N-dimethyl-N',N'-di(2-hydroxypropyl)propylenediamine.
[2] The curing agent for producing a polyurea resin according to the above [1], wherein in the formula (1), n is 0, and R is a hydroxymethyl group.
[3] The curing agent for producing a polyurea resin according to the above [1], wherein in the formula (1), n is 1, and R is a hydroxy group.
[4] An aqueous solution of the curing agent for producing a polyurea resin as defined in any one of the above [1] to [3].
[5] The aqueous solution of the curing agent for producing a polyurea resin according to the above [4], wherein the concentration of the diamine compound represented by the above formula (1) is from 0.5 to 30 wt%.
[6] A method for producing a polyurea resin, which comprises reacting a polyisocyanate compound and water in the presence of the curing agent for producing a polyurea resin as defined in any one of the above [1] to [3].
[7] A method for producing a polyurea resin, which comprises mixing a polyisocyanate compound and the aqueous solution of the curing agent for producing a polyurea resin as defined in the above [4] or [5], and heating the mixture.
[8] A polyurea resin, obtained by the production method as defined in the above [6] or [7].

Now, the present invention will be described in detail.

The curing agent for producing a polyurea resin of the present invention comprises at least one member selected from the group consisting of compounds each represented by the above formula (1).

As specific examples of the diamine compound of the above formula (1) wherein n is 0, the following compounds may be mentioned.

### EXEMPLARY COMPOUNDS

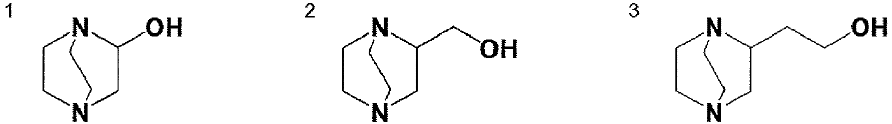

Further, as specific examples of the diamine compound of the above formula (1) wherein n is 1, the following compounds may be mentioned.

### EXEMPLARY COMPOUNDS

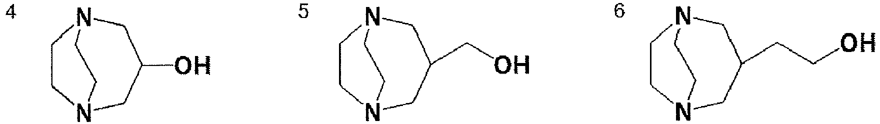

Among them, exemplary compounds 2 and 4 are particularly preferred as one component of the curing agent for producing a polyurea resin.

The diamine compound represented by the above formula (1) may be produced by a method as disclosed in JP-A-2012-149225.

The curing agent for producing a polyurea resin of the present invention comprises the diamine compound represented by the above formula (1) and at least one amine compound selected from the group consisting of N,N-dimethylaminoethanol, N,N-dimethylaminoisopropanol and N,N-dimethyl-N',N'-di(2-hydroxypropyl)propylenediamine, and may contain another amine compound within a range not to depart from the scope of the present invention. Such an amine compound may, for example, be a known tertiary amine. Specifically, it may, for example, be N,N-dimthyl-1,3-propanediamine, N-(3-aminopropyl)-N,N',N'-trimethyl-[2,2'-oxybis(ethaneamine)], N,N-dimethylaminoethanol, N,N-dimethylaminoisopropanol, N,N-dimethylhexanolamine, N,N-dimethylaminoethoxyethanol, N,N,N'-trimethylaminoethylethanolamine, 1,3,6,9-tetramethyl-3,6,9-triaza-1-decanol, N'-(2-hydroxypropyl)-N,N,N'-trimethyl-bis(2-aminoethyl)ether, N,N-dimethyl-N',N'-di(2-hydroxypropyl)propylenediamine or 1-(2-hydroxyethyl)-4-methylpiperazine. The content of such a diamine compound is not particularly limited, and is preferably within a range such that [the diamine compound represented by the above formula (1)]/[the amine compound]=99/1 to 70/30 (weight ratio).

A polyurea resin may be produced by reacting a polyisocyanate compound and water in the presence of the above curing agent of the present invention.

The polyisocyanate compound is not particularly limited and may, for example, be an aromatic polyisocyanate such as toluene diisocyanate (TDI), diphenylmethane diisocyanate (MDI), naphthylene diisocyanate or xylylene diisocyanate, an alicyclic polyisocyanate such as cyclohexyl diisocyanate or isophorone diisocyanate, an aliphatic polyisocyanate such as hexamethylene diisocyanate, a free isocyanate-containing prepolymer obtained by a reaction of such a polyisocyanate with a polyol, a modified polyisocyanate such as a carbodiimide-modified isocyanate, or a mixture thereof.

As TDI and its derivative, for example, a mixture of 2,4-TDI and 2,6-TDI, or a terminal isocyanate prepolymer derivative of TDI may be mentioned.

As MDI or its derivative, for example, a mixture of MDI and its polymer polyphenylpolymethylene diisocyanate, or a diphenylmethane diisocyanate derivative having a terminal isocyanate group may be mentioned.

Since the diamine compound represented by the above formula (1) is solid at room temperature, at the time of producing a polyurea resin, it is preferred to prepare an aqueous solution of the curing agent of the present invention, mix the aqueous solution with the polyisocyanate compound, and heat the mixture. Specifically, the polyisocyanate compound and the aqueous solution of the curing agent of the present invention are applied to the surface of one of adherends to form an adhesive layer (adhesive sheet), the other adherend is overlaid on the adhesive layer, followed by heating and curing, whereby the adherends are bonded.

In the aqueous solution of the curing agent of the present invention, the concentration of the diamine compound represented by the above formula (1) is not particularly limited, and is preferably within a range of from 0.1 to 50 wt%, more preferably from 0.5 to 30 wt%.

Further, the aqueous solution of the curing of the present invention may contain an organic solvent within a range not to depart from the scope of the present invention. That is, the diamine compound represented by the above formula (1) may be dissolved in an organic solvent, and then water is added to prepare an aqueous solution of the curing agent of the present invention.

Such an organic solvent may, for example, be propylene glycol, dipropylene glycol, ethylene glycol, diethylene glycol or 1,4-butanediol. Considering the adhesive strength of the polyurea resin to be obtained, the concentration of the organic solvent in the aqueous solution of the curing agent of the present invention is preferably at most 10 wt%.

### ADVANTAGEOUS EFFECTS OF INVENTION

A polyurea resin obtained by using the curing agent for producing a polyurea resin of the present invention is capable of thermal compression molding at the time of producing a synthetic board or at the time of producing a ceiling material for an automobile, which is free from the odor and toxicity or environmental problems at the time of the molding or of products, which has favorable adhesive strength, and which causes substantially no pollution of other materials, and is thereby industrially very useful.

### EXAMPLES

Now, the present invention will be described with reference to Examples and Comparative Examples. However, it should be understood that the present invention is by no means restricted to such specific Examples.

### PREPARATION EXAMPLE 1: Preparation of exemplary compounds 2 and 4

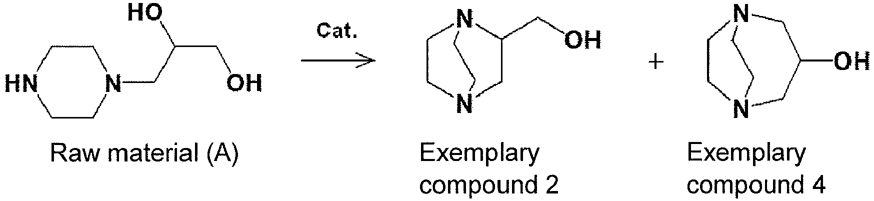

A center portion of a quartz glass tube having an internal diameter of 40 mm was filled with 160 ml of a catalyst for a gaseous phase reaction (the catalyst for a gaseous phase reaction as disclosed in Reference Example 1 of JP-A-2012-149225 was used), and the upper and lower parts thereof were filled with a Raschig ring having an outer diameter of 5 mm. The catalyst layer and the Raschig ring layers were kept at 360°C by an electric furnace, and an aqueous solution (2 mol%) of 1.6 kg (10 mol) of the raw material (A) was dropwise added from the top of the tube at a rate of GHSV=1,500 Hr⁻¹. Further, a nitrogen gas as a diluent gas was entrained at GHSV=750 Hr⁻¹. 3 Hours after initiation of passage of the aqueous solution, the reaction liquid was collected over a period of 1 hour and analyzed by gas chromatography and as a result, the degree of conversion was 100%. The obtained components were analyzed by GC-MS, isolated by precision distillation and analyzed by NMR and elemental analysis, and found to be the above exemplary compounds 2 and 4, and two types of diamine compound were obtained.

### PREPARATION EXAMPLE 2: Preparation of 1,3,6,9-tetramethyl-3,6,9-triaza-1-decanol (TMTAD)

Into a stainless steel autoclave having a capacity of 1 liter (hereinafter referred to as "reactor 1"), 413 g of diethylenetriamine (4 mol) was charged. The reactor 1 was replaced with nitrogen by purging, and 116 g of propylene oxide (2 mol) was added by a pump over a period of 3 hours at 80°C under 0.6 MPa, and the reactor 1 was cooled to 25°C to obtain a reaction liquid.

Then, into a stainless steel autoclave having a capacity of 0.2 liter (hereinafter referred to as "reactor 2"), 35 g of the reaction liquid obtained by the above reaction, 35 g of water and 0.11 g of palladium carbon catalyst were charged. The reactor 2 was replaced with nitrogen and hydrogen by purging, heated to 130°C and pressurized with hydrogen to 3.0 MPa. While the temperature and the pressure were maintained, 65.5 g of a 37% aqueous formaldehyde solution was supplied to the reactor 2 by a pump over a period of from 3 to 4 hours, and the reaction was continued further for 1 hour. The catalyst was removed by filtration from the obtained reaction liquid, vacuum distillation (column top temperature: 120 to 130°C, degree of pressure reduction: 100 mmHg) was carried out to remove methanol, water and low molecular weight byproducts, and further vacuum reduction (column top temperature; 143°C, degree of pressure reduction: 28 mmHg) was carried out to obtain 28 g of 1,3,6,9-tetramethyl-3,6,9-triaza-1-decanol.

### PREPARATION EXAMPLE 3: Preparation of N-(3-aminopropyl)-N,N',N'-trimethyl-[2,2'-oxybis(ethaneamine)] (APTMOBEA)

Into a stainless steel autoclave having a capacity of 2 liters (hereinafter referred to as "reactor 3"), 499 g of dimethylaminoethoxy ethanol (3.8 mol) and 38 g of copper/zinc oxide/alumina catalyst were charged. The reactor 3 was replaced with nitrogen and hydrogen by purging, and the catalyst was subjected to reduction treatment in the system over a period of 9 hours under 5.6 MPa at 195°C. The reactor 3 was cooled to 25°C and depressurized to atmospheric pressure, and 177 g of monomethylamine (5.7 mol) was injected utilizing nitrogen pressure of 0.7 MPa. The pressure in the reactor 3 was elevated to 1.5 MPa again with hydrogen, the temperature was increased to 195°C, and the temperature and the pressure were maintained for 24 hours. The obtained reaction liquid was subjected to distillation to completely remove low boiling point compounds, high boiling point compounds and very small amounts of components derived from the copper/zinc oxide/alumina catalyst. The main component in 326 g of the product collected by distillation was N,N,N'-trimethylbis(aminoethyl)ether and dimethylaminoethoxy ethanol.

Then, 326 g of the product collected by distillation was charged to a three-necked flask equipped with a reflux condenser and heated to 55°C, and 71 g of acrylonitrile (1.3 mol) was charged over a period of 2 hours. The reaction was continued further for 5 hours to reduce unreacted N,N,N'-trimethylbis(aminoethyl)ether to at most 1%.

Then, into the reactor 1, 20 g of a chromium-facilitated sponge nickel catalyst and 150 g of a 28% aqueous ammonium hydroxide solution were charged. The reactor 1 was replaced with nitrogen and hydrogen by purging, heated to 90°C and pressurized with hydrogen to 8.2 MPa. 326 g of the reaction liquid obtained by the above reaction was supplied to the reactor 2 by a pump over a period of 3.5 hours, and the reaction was continued further for 1 hour. The catalyst was removed by filtration from the obtained reaction liquid, and vacuum distillation (column top temperature: 124 to 133°C, degree of pressure reduction: 13 hPa) was carried out to obtain 140 g of N-(3-aminoprpyl)-N,N',N'-trimethyl-[2,2'-oxybis(ethaneamine)].

### EXAMPLES 1 to 3, COMPARATIVE EXAMPLES 1 to 15 and REFERENCE EXAMPLES 1 to 13

Using as a curing agent amine compounds as identified in Tables 1 and 2, they were formed into aqueous solutions having optional concentrations, and the adhesive strength and polyvinyl chloride sheet (PVC) staining properties were evaluated. The results are shown in Tables 3 to 5. In Tables 2 and 5, HC-3 to HC-14 correspond to reactive amine catalysts, HC-3 and HC-11 are ones having a primary amino group as a reactive group, HC-5, HC-6, HC-8, HC-9, HC-10 and HC-12 are ones having a primary hydroxy group as a reactive group, HC-4 and HC-7 are ones having a secondary hydroxy group as a reactive group, and HC-13 and HC-14 are a blend thereof.

**[Table 1]**

| Curing agent | Composition and weight ratio of curing agent |
|---|---|
| C-1 | Amine compound (1)^{a)}/amine compound (2)^{b)}=100/0 |
| C-2 | Amine compound (1)/amine compound (2)=95/5 |
| C-3 | Amine compound (1)/amine compound (2)=90/10 |
| C-4 | Amine compound (1)/amine compound (2)=80/20 |
| C-5 | Amine compound (1)/amine compound (2)=65/35 |
| C-6 | Amine compound (1)/amine compound (2)=0/100 |
| C-7 | C-3/DMAPA-2PO=70/30 |
| C-8 | C-3/CMEA=70/30 |
| C-9 | C-3/DMIPA=70/30 |

| | |
|---|---|
| Amine compound (1)^{a)}: Exemplary compound 2 (synthetic product, Preparation Example 1) Amine compound (1)^{b)}: Exemplary compound 5 (synthetic product, Preparation Example 1) DMAPA-2PO: N,N-dimethyl-N',N'-di(2-hydroxypropyl)propylenediamine (manufactured by Sigma-Aldrich) DMEA: N,N-dimethylaminoethanol (manufactured by Tokyo Chemical Industry Co., Ltd.) DMIPA: N,N-dimethylaminoisopropanol (manufactured by Tokyo Chemical Industry Co., Ltd.) | |

**[Table 2]**

| Curing agent | Composition and weight ratio of curing agent |
|---|---|
| HC-1 | BDMEE¹⁾ |
| HC-2 | TEDA²⁾ |
| HC-3 | DMAPA³⁾ |
| HC-4 | DMAPA-2PO⁴⁾ |
| HC-5 | TMAEEA³⁾ |
| HC-6 | DMAEE⁶⁾ |
| HC-7 | TMTAD⁷⁾ |
| HC-8 | MHEP⁸⁾ |
| HC-9 | DMEA⁹⁾ |
| HC-10 | HPTMBAEE¹⁰⁾ |
| HC-11 | APTMOBEA¹¹⁾ |
| HC-12 | DMHA¹²⁾ |
| HC-13 | DMIPA¹³⁾ |
| HC-14 | DMAPA/DMAPA-2PO= 5/1 |
| HC-15 | DMAPA/TMAEEA =1/1 |
| HC-16 | C-3/HPTMBAEE=70/30 |

| | |
|---|---|
| BDMEE¹⁾: Bis(2-dimethylaminoethyl) ether (TOYOCAT-ETS manufactured by Tosoh Corporation) TEDA²⁾: Triethylenediamine (TEDA manufactured by Tosoh Corporation) DMAPA³⁾: N,N-dimethyl-1,3-propanediamine (manufactured by Tokyo Chemical Industry Co., Ltd.) DMAPA-2PO⁴⁾: N,N-dimethyl-N',N'-di(2-hydroxypropyl)propylenediamine (manufactured by Sigma-Aldrich) TMAEEA⁵⁾: N,N,N'-trimethylaminoethylethanolamine (TOYOCAT-RX5 manufactured by Tosoh Corporation) DMAEE⁶⁾: N,N-dimethylaminoethoxyethanol (manufactured by Tokyo Chemical Industry Co., Ltd.) TMTAD⁷⁾: 1,3,6,9-Tetramethyl-3,6,9-triaza-1-decanol (synthetic product, Preparation Example 2) MHEP⁸⁾: 1-(2-Hyroxyethyl)-4-methylpiperazine (manufactured by Wako Pure Chemical Industries, Ltd.) DMEA⁹⁾: N,N-dimethylaminoethanol (manufactured by Tokyo Chemical Industry Co., Ltd.) HPTMBAEE¹⁰⁾: N'-(2-hydroxypropyl)-N,N,N'-trimethyl-bis(2-aminoethyl)ether (JEFFCAT-ZF10 manufactured by Hantsman) APTMOBEA¹¹⁾: N-(3-aminopropyl)-N,N',N'-trimethyl-[2,2'-oxybis(ethaneamine)] (synthetic product, Preparation Example 3) DMHA¹²⁾: N,N-dimethylaminohexanol (manufactured by Tokyo Chemical Industry Co., Ltd.) DMIPA¹³⁾: N,N-dimethylaminoisopropanol (manufactured by Tokyo Chemical Industry Co., Ltd.) | |

**[Table 3]**

| | | Examples | | | Reference Examples | | | | | | |
|---|---|---|---|---|---|---|---|---|---|---|---|
| | | 1 | 2 | 3 | 1 | 2 | 3 | 4 | 5 | 6 | 7 |
| Adhesion test (canvas/canvas) | | | | | | | | | | | |
| Isocyanate | (g/100cm²) | 2.24 | 2.29 | 2.27 | | 2.29 | 2.30 | 2.32 | 2.25 | 2.29 | 2.28 |
| [2 wt% aqueous solution] | | | | | | | | | | | |
| C-1 | (g/100cm²) | | | | | 0.61 | | | | | |
| C-2 | (g/100cm²) | | | | | | 0.58 | | | | |
| C-3 | (g/100cm²) | | | | | | | 0.62 | | | |
| C-4 | (g/100cm²) | | | | | | | | 0.50 | | |
| C-5 | (g/100cm²) | | | | | | | | | 0.60 | |
| C-6 | (g/100cm²) | | | | | | | | | | 0.49 |
| C-7 | (g/100cm²) | 0.55 | | | | | | | | | |
| C-8 | (g/100cm²) | | 0.51 | | | | | | | | |
| C-9 | (g/100cm²) | | | 0.56 | | | | | | | |
| Adhesion strength | | | | | | | | | | | |
| T-peel strength | (* 10²g/25mm) | 9.4 | 9.3 | 9.1 | | 8.5 | 8.2 | 9.0 | 7.9 | 8.6 | 8.8 |
| PVC staining properties | | | | | | | | | | | |
| Isocyanate | (g) | 11.47 | 11.47 | 11.47 | | 11.47 | 11.47 | 11.47 | 11.47 | 11.47 | 11.47 |
| [5 wt% aqueous solution] | | | | | | | | | | | |
| C-1 | (g) | | | | | 1.00 | | | | | |
| C-2 | (g) | | | | | | 1.00 | | | | |
| C-3 | (g) | | | | | | | 1.00 | | | |
| C-4 | (g) | | | | | | | | 1.00 | | |
| C-5 | (g) | | | | | | | | | 1.00 | |
| C-6 | (g) | | | | | | | | | | 1.00 |
| C-7 | (g) | 1.00 | | | | | | | | | |
| C-8 | (g) | | 1.00 | | | | | | | | |
| C-9 | (g) | | | 1.00 | | | | | | | |
| Urea sheet weight | (g) | 2.70 | 2.74 | 2.71 | | 2.74 | 2.73 | 2.75 | 2.70 | 2.74 | 2.72 |
| Color difference ΔE | 90°C×72 hr | 0.5 | 0.6 | 0.6 | 0.4 | 0.5 | 0.5 | 0.5 | 0.5 | 0.6 | 0.6 |
| | 90°C×168 hr | 0.8 | 0.9 | 0.9 | 0.7 | 0.9 | 1.0 | 0.9 | 0.9 | 1.0 | 1.1 |

**[Table 4]**

| | | Reference Examples | | | | | |
|---|---|---|---|---|---|---|---|
| | | 4 | 8 | 9 | 10 | 11 | 12 |
| Adhesion test (canvas/canvas) | | | | | | | |
| Isocyanate | (g/100cm²) | 2.32 | 2.29 | 2.30 | 2.33 | 2.38 | 2.31 |
| [Aqueous solution concentration] | | 2% | 10% | 20% | 30% | 50% | 50% |
| C-3 | (g/100cm²) | 0.62 | 0.58 | 0.53 | 0.49 | 0.45 | 0.68 |
| Adhesion strength | | | | | | | |
| T-peel strength (*10²g/25mm) | | 9.0 | 8.2 | 7.5 | 7.1 | 4.3 | 9.2 |
| PVC staining properties | | | | | | | |
| Isocyanate | (g) | 11.47 | 11.47 | 11.47 | 11.47 | 11.47 | 11.47 |
| [Aqueous solution concentration] | | 5% | 10% | 20% | 30% | 50% | 50% |
| C-3 | (g) | 1.00 | 1.00 | 1.00 | 1.00 | 1.00 | 1.40 |
| Urea sheet weight | (g) | 2.75 | 2.71 | 2.74 | 2.75 | 2.73 | 2.72 |
| Color difference ΔE | 90°C×72 hr | 0.5 | 0.6 | 0.7 | 0.7 | 0.6 | 1.0 |
| | 90°C×168 hr | 0.9 | 1.0 | 1.0 | 1.2 | 1.1 | 1.6 |

**[Table 5]**

| | | Comparative Examples | | | | | | | | | | | | | | | R.E. |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 13 |
| Adhesion test (canvas/canvas) | | | | | | | | | | | | | | | | | |
| Isocyanate | (g / 100cm²) | 2.33 | 2.23 | 2.34 | 2.27 | 2.26 | 2.28 | 2.27 | 2.26 | 2.33 | 2.31 | 2.22 | 2.25 | 2.28 | 2.20 | 2.23 | 2.31 |
| [2 wt% aqueous solution] | | | | | | | | | | | | | | | | | |
| HC-1 | (g/100cm²) | 0.52 | | | | | | | | | | | | | | | |
| HC-2 | (g/100cm²) | | 0.53 | | | | | | | | | | | | | | |
| HC-3 | (g/100cm²) | | | 0.47 | | | | | | | | | | | | | |
| HC-4 | (g/100cm²) | | | | 0.40 | | | | | | | | | | | | |
| HC-5 | (g/100cm²) | | | | | 0.51 | | | | | | | | | | | |
| HC-6 | (g/100cm²) | | | | | | 0.68 | | | | | | | | | | |
| HC-7 | (g/100cm²) | | | | | | | 0.55 | | | | | | | | | |
| HC-8 | (g/100cm²) | | | | | | | | 0.56 | | | | | | | | |
| HC-9 | (g/100cm²) | | | | | | | | | 0.64 | | | | | | | |
| HC-10 | (g/100cm²) | | | | | | | | | | 0.54 | | | | | | |
| HC-11 | (g/100cm²) | | | | | | | | | | | 0.56 | | | | | |
| HC-12 | (g/100cm²) | | | | | | | | | | | | 0.57 | | | | |
| HC-13 | (g/100cm²) | | | | | | | | | | | | | 0.56 | | | |
| HC-14 | (g/100cm²) | | | | | | | | | | | | | | 0.52 | | |
| HC-15 | (g/100cm²) | | | | | | | | | | | | | | | 0.53 | |
| HC-16 | (g/100cm²) | | | | | | | | | | | | | | | | 0.55 |
| Adhesion strength | | | | | | | | | | | | | | | | | |
| T-peel strength(*10²g/25mm) | | 12.9 | 8.1 | 1.4 | 7.7 | 7.8 | 10.9 | 9.3 | 8.3 | 9.1 | 9.6 | 4.8 | 4.7 | 5.3 | 1.6 | 2.8 | 8.9 |
| PVC staining properties | | | | | | | | | | | | | | | | | |
| Isocyanate | (g) | 11.47 | 11.47 | 11.47 | 11.47 | 11.47 | 11.47 | 11.47 | 11.47 | 11.47 | 11.47 | 11.47 | 11.47 | 11.47 | 11.47 | 11.47 | 11.47 |
| [5 wt% aqueous solution] | | | | | | | | | | | | | | | | | |
| HC-1 | (g) | 1.00 | | | | | | | | | | | | | | | |
| HC-2 | (g) | | 1.00 | | | | | | | | | | | | | | |
| HC-3 | (g) | | | 1.00 | | | | | | | | | | | | | |
| HC-4 | (g) | | | | 1.00 | | | | | | | | | | | | |
| HC-5 | (g) | | | | | 1.00 | | | | | | | | | | | |
| HC-6 | (g) | | | | | | 1.00 | | | | | | | | | | |
| HC-7 | (g) | | | | | | | 1.00 | | | | | | | | | |
| HC-8 | (g) | | | | | | | | 1.00 | | | | | | | | |
| HC-9 | (g) | | | | | | | | | 1.00 | | | | | | | |
| HC-10 | (g) | | | | | | | | | | 1.00 | | | | | | |
| HC-11 | (g) | | | | | | | | | | | 1.00 | | | | | |
| HC-12 | (g) | | | | | | | | | | | | 1.00 | | | | |
| HC-13 | (g) | | | | | | | | | | | | | 1.00 | | | |
| HC-14 | (g) | | | | | | | | | | | | | | 1.00 | | |
| HC-15 | (g) | | | | | | | | | | | | | | | 1.00 | |
| HC-16 | (g) | | | | | | | | | | | | | | | | 1.00 |
| Urea sheet weight | | 2.74 | 2.73 | 2.71 | 2.70 | 2.72 | 2.71 | 2.72 | 2.75 | 2.74 | 2.74 | 2.72 | 2.73 | 2.74 | 2.73 | 2.75 | 2.73 |
| Color difference ΔE | ** | 6.4 | 2.0 | 2.2 | 0.8 | 2.8 | 2.7 | 3.1 | 1.8 | 1.4 | 3.6 | 2.4 | 1.0 | 1.5 | 1.5 | 1.4 | 1.6 |
| | *** | 14.5 | 5.1 | 4.5 | 1.4 | 5.3 | 5.8 | 5.4 | 3.4 | 2.4 | 9.2 | 7.9 | 1.9 | 2.8 | 3.0 | 2.4 | 3.1 |

| | | | | | | | | | | | | | | | | | |
|---|---|---|---|---|---|---|---|---|---|---|---|---|---|---|---|---|---|
| ** 90°C×72hr R.E.: Reference Example *** 90°C×168hr | | | | | | | | | | | | | | | | | |

In Examples, Comparative Examples and Reference Examples, the methods for evaluating the adhesive strength and the polyvinyl chloride sheet (PVC) staining properties were as follows.

### [Method for evaluating adhesive strength of polyurea resin]

### (1) Preparation of adhesive test sample and measurement of adhesive strength

As canvas as an adherend, No. 9 canvas was used and two sheets having a size of 15 cm × 10 cm were cut. To one of the two sheets, polyisocyanate (polymeric MDI manufactured by Nippon Polyurethane Industry Co., Ltd., tradename: MR-200, NCO content: 30.9%) was applied with a glass rod (diameter: 15 mm) so that the application amount would be 2.2 to 2.5 g/100 cm² (10 cm × 10 cm). Then, an aqueous solution containing 5 wt% of a curing agent as identified in Table 1 or 2 was applied by using a spray gun (manufactured by MEIJI AIR COMPRESSOR MFG. CO., LTD., model: F75) for 10 seconds. The application amount was within a range of from 0.40 to 0.68 g/100 cm². Then, the other sheet of canvas was overlaid on the canvas coated with the polyisocyanate and the aqueous solution of the curing agent, and immediate subjected to thermal compression bonding by a manual pressing machine (manufactured by SHINTO Metal Industries Corporation) the temperature of which was adjusted to 140°C, under 1.5 MPa for 22 seconds. After cooling, three strip samples having a width of 25 mm and a length of 150 mm were obtained from one obtained laminate of canvas/adhesive layer/canvas by a punching machine. Each sample was cured in a constant temperature chamber (23°C, 50% humidity) for 24 hours and subjected to a T-peel test by using a tensile testing machine (manufactured by ORIENTEC CORPORATION, model: RTM-500) to measure the adhesive strength.

### [Method for evaluating polyvinyl chloride sheet (PVC) staining properties]

### (1) Method for preparing polyvinyl chloride sheet (PVC) for staining test

36 g of white polyvinyl chloride compound (manufactured by PLAS-TECH CORPORATION, tradename: POLYVIN compound) was put in a mold (12 cm × 12 cm × 1.5 mm) and preheated for 5 minutes by a manual pressing machine set at 170°C, removal of air was conducted four times, and the compound was pressed under a pressure (20 MPa) for 5 minutes. The compound was cooled for 10 minutes while being pressurized (15 MPa) by a cooling pressing machine set at 10°C to complete a polyvinyl chloride sheet (PVC). For the staining test, a sample of 35 mm × 35 mm × 1.5 mm was obtained from the white sheet.

### (2) Method for preparing urea sheet for staining test

In a 200 cc PE cup, 11.47 g of isocyanate (MR-200) and 1 g of a 5 wt% aqueous solution of a curing agent as identified in Table 3 or 4 were added and stirred at room temperature by a glass rod. When the liquid was thickened and formed into a putty, the liquid was filled in a Teflon (registered trademark) mold (60 mm × 50 mm × 3 mm). As the mold, a polyester film was overlaid at the bottom, then a Teflon (registered trademark) sheet was placed and a frame was placed thereon. After the sample was filled, a Teflon (registered trademark) sheet and a polyester film were put in this order, and the sample was pressed by a manual pressing machine the temperature of which was adjusted to 140°C for 40 seconds (completion of emission of water vapor was confirmed). The pressurization condition was such that the pressing machine gauge pressure=0 MPa (to an extent of press adhesion). The urea sheet molded by the above method was cut into a width of 35 to 40 mm, and the length was adjusted so that the weight would be 2.70 to 2.75 g.

### (3) Method of test on PVC staining properties

The urea sheet prepared by the method for preparing urea sheet for staining test was put in a 140 cc sample bottle, the white polyvinyl chloride sheet (PVC) was placed thereon, heat history was applied at 90°C for 72 hours or at 90°C for 168 hours, and the degree of staining (discoloration) of the polyvinyl chloride sheet (PVC) was measured by a colorimeter (manufactured by NIPPON DENSHOKU INDUSTRIES CO., LTD., Color meter ZE6000).

As evident from Examples 1 to 3 in Table 3, a polyurea resin obtained by using the curing agent of the present invention by thermal compression molding has sufficient adhesive strength. Further, the color difference of PVC in Reference Example 1 indicates discoloration only by the heat history, and the polyurea resins in Examples 1 to 3 were found to have very small PVC staining properties also by comparison with the color difference in Reference Example 1. Accordingly, it is understood that the PVC staining properties when the polyurea resin is used for a synthetic board or for a ceiling material for an automobile can be minimized.

In Reference Examples 2 to 7 in Table 3, the composition ratio of the exemplary compounds 2 and 4 as the compound represented by the above formula (1) was changed, and the PVC staining properties were at the same level as Examples, but the adhesive strength is somewhat poor. In Reference Examples 4 and 8 to 12 in Table 4, the composition ratio of the exemplary compounds 2 and 4 as the compound represented by the above formula (1) of 90/10 and the content of water were changed. If the concentration is too high, the adhesive strength decreases when the aqueous solution concentration is low as shown in Reference Example 11, and on the contrary, the PVC staining properties tend to be significant if the aqueous solution concentration is high as shown in Reference Example 12. In Reference Example 13 in Table 5, exemplary compounds 2 and 4 as the compound represented by the above formula (1) in a composition ratio of 90/10 and an amino alcohol other than N,N-dimethylaminoethanol, N,N-dimethylaminoisopropanol, N,N-dimethyl-N',N'-di(2-hydroxypropyl)propylenediamine were blended in a ratio of 70/30, and the PVC staining properties were poor.

In Comparative Examples 1 to 15 in Table 5, the PVC staining properties were poor as compared with Examples. Further, in Comparative Examples 3 and 11 to 14, the adhesive strength was poor. As mentioned above, it is found that the effects of the present invention will not be obtained by a conventional reactive amine catalyst.

The present invention has been described in detail with reference to specific embodiments, and it is obvious for the person skilled in the art that various changes and modifications are possible without departing from the intention and the scope of the present invention.

The entire disclosure of Japanese Patent Application No. 2014-084842 filed on April 16, 2014 including specification, claims, drawings and summary is incorporated herein by reference in its entirety.

### INDUSTRIAL APPLICABILITY

The polyurea resin obtained by using the curing agent of the present invention is useful as an adhesive in production of a synthetic board or a ceiling material for an automobile.

## Claims

1. A curing agent for producing a polyurea resin, which comprises at least one diamine compound selected from the group consisting of compounds each represented by the following formula (1): 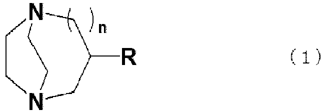 wherein n is 0 or 1, and R is a hydroxy group or hydroxyalkyl group having 0 to 2 carbon atoms, and at least one amine compound selected from the group consisting of N,N-dimethylaminoethanol, N,N-dimethylaminoisopropanol and N,N-dimethyl-N',N'-di(2-hydroxypropyl)propylenediamine.

2. The curing agent for producing a polyurea resin according to Claim 1, wherein in the formula (1), n is 0, and R is a hydroxymethyl group.

3. The curing agent for producing a polyurea resin according to Claim 1, wherein in the formula (1), n is 1, and R is a hydroxy group.

4. An aqueous solution of the curing agent for producing a polyurea resin as defined in any one of Claims 1 to 3.

5. The aqueous solution of the curing agent for producing a polyurea resin according to Claim 4, wherein the concentration of the diamine compound represented by the formula (1) as defined in Claim 1 is from 0.5 to 30 wt%.

6. A method for producing a polyurea resin, which comprises reacting a polyisocyanate compound and water in the presence of the curing agent for producing a polyurea resin as defined in any one of Claims 1 to 3.

7. A method for producing a polyurea resin, which comprises mixing a polyisocyanate compound and the aqueous solution of the curing agent for producing a polyurea resin as defined in Claim 4 or 5, and heating the mixture.

8. A polyurea resin, obtained by the production method as defined in Claim 6 or 7.
